(19) Europäisches Patentamt
European Patent Office
Office européen des brevets

(11) Veröffentlichungsnummer: **0 450 180 A2**

# (12) EUROPÄISCHE PATENTANMELDUNG

(21) Anmeldenummer: **90124869.0**

(22) Anmeldetag: **20.12.90**

(51) Int. Cl.5: **C07D 213/61**, //C07D213/50, C07D413/04

(30) Priorität: **03.03.90 DE 4006794**

(43) Veröffentlichungstag der Anmeldung: **09.10.91 Patentblatt 91/41**

(84) Benannte Vertragsstaaten: **BE CH DE DK ES FR GB IT LI NL**

(71) Anmelder: **BAYER AG**
**W-5090 Leverkusen 1 Bayerwerk(DE)**

(72) Erfinder: **Gallenkamp, Bernd, Dr.**
**Paul-Ehrlich-Strasse 13**
**W-5600 Wuppertal 1(DE)**

## (54) Verfahren zur Herstellung von Aminoethanolderivaten.

(57) Die vorliegende Erfindung betrifft ein neues Verfahren zur Herstellung von teilweise bekannten Aminoethanolderivaten,
der allgemeinen Formel (I)

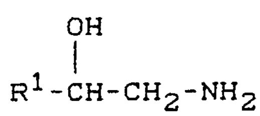

(I)

in welcher
$R^1$ für gegebenenfalls substituiertes Pyridyl steht,
das dadurch gekennzeichnet ist, daß man Aminomethylketone der allgemeinen Formel (II)

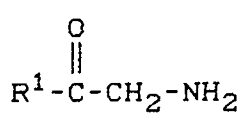

(II)

in welcher
$R^1$ die oben angegebene Bedeutung hat,
oder Säureaddukte von Aminomethylketonen der Formel (II) mit Hydrierungsmitteln umsetzt, sowie neue Pyridylaminomethylketone der allgemeinen Formel (IIa)

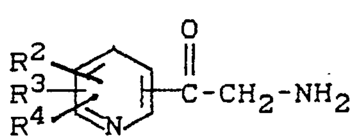

(IIa)

in welcher
$R^2$, $R^3$, $R^4$ die in der Beschreibung angegebene Bedeutung haben, und deren Herstellung.

EP 0 450 180 A2

Die vorliegende Erfindung betrifft ein neues Verfahren zur Herstellung von teilweise bekannten Aminoethanolderivaten, neue Zwischenprodukte hierfür und Verfahren zu deren Herstellung.

Es ist bekannt, daß man Aminoethanolderivate durch Reduktion entsprechender Nitroethanolderivate erhalten kann (vgl. Arch Pharm. 291 (1958), 12-22; loc. cit. 292 (1959), 496-508; loc. cit. 297 (1964), 10-30. Zur Herstellung der hierfür als Ausgangsstoffe benötigten Nitroethanolderivate wird jedoch Nitromethan benötigt, dessen Verwendung aus sicherheitstechnischen Gründen für industrielle Belange sehr problematisch ist.

Weiter ist bekannt, daß Aminoethanolderivate auch durch Umsetzung von Halogenethanolderivaten mit Ammoniak synthetisiert werden können (vgl. Chem. Abstracts Zitat 60 (1964), 6815c); EP-A 244 728).

Die Ausbeute und die Qualität der hierbei erhaltenen Produkte sind jedoch meist unbefriedigend.

Gegenstand der vorliegenden Erfindung sind

1. ein Verfahren zur Herstellung von Aminoethanolderivaten der allgemeinen Formel (I)

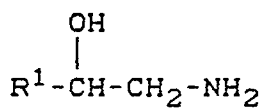

(I)

in welcher

R für gegebenenfalls substituiertes Pyridyl steht,

dadurch gekennzeichnet, daß man Aminomethylketone der allgemeinen Formel (II)

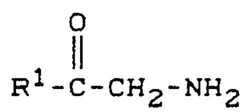

(II)

in welcher

$R^1$ die oben angegebene Bedeutung hat,

oder Säureaddukte von Aminomethylketonen der Formel (II) mit Hydrierungsmitteln gegebenenfalls in Gegenwart von Reaktionshilfsmitteln und gegebenenfalls in Gegenwart von Verdünnungsmitteln bei Temperaturen zwischen -50 °C und +150 °C umsetzt;

2. neue Pyridylaminomethylketone der allgemeinen Formel (IIa)

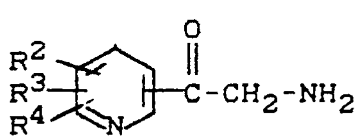

(IIa)

in welcher

$R^2$ für Halogen oder CN steht,

$R^3$ für H, Halogen oder $NH_2$ steht,

$R^4$ für H oder Halogen steht

und Säureaddukte von Pyridylaminomethylketonen der Formel (IIa);

3. ein Verfahren zur Herstellung von Aminomethylketonen der allgemeinen Formel (II)

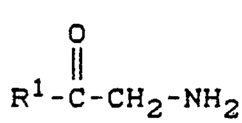

(II)

in welcher

$R^1$ für gegebenenfalls substituiertes Pyridyl steht,

oder von Säureaddukten von Aminomethylketonen der Formel (II)

dadurch gekennzeichnet, daß man Oxazolcarbonsäureester der allgemeinen Formel (III)

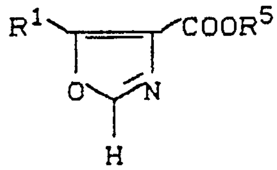

(III)

in welcher

$R^1$ die oben angegebene Bedeutung hat und

$R^5$ für Niederalkyl steht,

mit Säuren bei Temperaturen zwischen 50 °C und 100 °C in Gegenwart eines Verdünnungsmittels umsetzt, und gegebenenfalls die Produkte anschließend durch Behandeln mit starken Basen freisetzt;

4. neue Oxazolcarbonsäureester der allgemeinen Formel (IIIa)

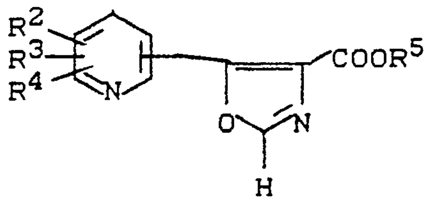

(IIIa)

in welcher

$R^2$, $R^3$, $R^4$ die oben angegebene Bedeutung haben,

$R^5$ für Niederalkyl steht,

5. Verfahren zur Herstellung von Oxazolcarbonsäureestern der allgemeinen Formel (III)

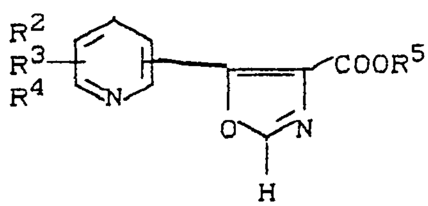

(III)

in welcher

$R^2$, $R^3$, $R^4$, $R^5$ die oben angegebene Bedeutung haben,

dadurch gekennzeichnet, daß man Carbonsäurechloride der allgemeinen Formel (IV)

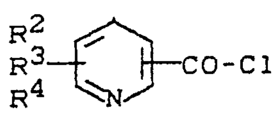

(IV)

in welcher

$R^2$, $R^3$, $R^4$ die oben angegebene Bedeutung haben,

mit Isocyanessigsäureestern der allgemeinen Formel (V)

$CN\text{-}CH_2\text{-}COOR^5$ (V)

in welcher

$R^5$ die oben angegebene Bedeutung hat

in Gegenwart eines Säureakzeptors und gegebenenfalls in Gegenwart eines Verdünnungsmittels bei Temperaturen zwischen -20 ° C und +50 ° C umsetzt.

Der Reaktionsablauf bei den erfindungsgemäßen Verfahrensschritten kann beispielsweise durch das folgende Formelschema skizziert werden:

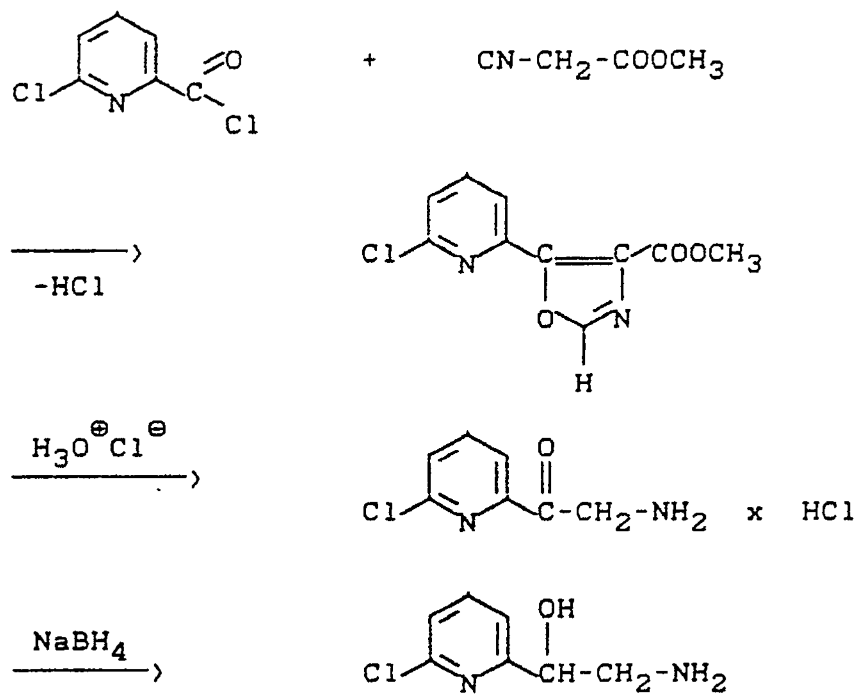


In den Formeln (I) und (II) steht $R^1$ vorzugsweise für 2-Pyridyl, 3-Pyridyl oder 4-Pyridyl, welche jeweils gegebenenfalls einfach bis vierfach (insbesondere einfach oder zweifach) substituiert sind durch gleiche oder verschiedene Reste aus der Reihe Halogen (insbesondere Fluor, Chlor, Brom), $C_1$-$C_4$-Alkyl (insbesondere Methyl, Ethyl), $C_1$-$C_4$-Halogenalkyl (insbesondere Trifluormethyl), $C_1$-$C_4$-Alkoxy (insbesondere Methoxy, Ethoxy), $C_1$-$C_4$-Halogenalkoxy (insbesondere Difluormethoxy, Trifluormethoxy), CN, Amino, $C_{1-4}$-Mono- oder -Dialkylamino, Pyrrolo, Acylamin, Hydroxyl, $C_{1-4}$-Hydroxyalkyl, $C_{1-4}$-Alkylcarbonyloxy.

In den Formeln (I) und (II) steht $R^1$ insbesondere für 2-Pyridyl, 3-Pyridyl oder 4-Pyridyl, durch Fluor, Chlor, Brom, CN, $NH_2$ substituiert sind.

Säureaddukte von Verbindungen der Formel (II) sind vorzugsweise die Addukte mit Hydrogenhalogeniden, wie z.B. mit Hydrogenchlorid, Hydrogenbromid und Hydrogeniodid, insbesondere die Hydrogenchlorid-Addukte.

Als Beispiele für die Verbindungen der Formel (I) seien genannt:

2-(2-Amino-1-hydroxy-ethyl)-pyridin, 3-Chlor-, 4-Chlor-, 5-Chlor-, 6-Chlor-, 3,4-Dichlor-, 3,5-Dichlor-, 3,6-Dichlor-, 4,5-Dichlor-, 4,6-Dichlor- und 5,6-Dichlor-2-(2-amino-1-hydroxy-ethyl)-pyridin, 3-(2-Amino-1-hydroxy-ethyl)-pyridin, 2-Chlor-, 4-Chlor-, 5-Chlor-, 6-Chlor-, 2,4-Dichlor-, 2,5-Dichlor-, 2,6-Dichlor-, 4,5-Dichlor-, 4,6-Dichlor- und 5,6-Dichlor-3-(2-amino-1-hydroxy-ethyl)-pyridin, 4-(2-Amino-1-hydroxy-ethyl)-pyridin, 2-Chlor-, 3-Chlor-, 2,3-Dichlor-, 2,5-Dichlor-, 2,6-Dichlor- und 3,6-Dichlor-4-(2-amino-1-hydroxy-ethyl)-pyridin.

Die Aminoethanolderivate der Formel (I) sind bekannt (vgl. oben zitierter Stand der Technik sowie EP-A 305845 und DE-P 3 902 286 vom 26.01.1989).

Als Beispiele für die Verbindungen der Formel (II) seien genannt:

2-Aminoacetyl-pyridin, 3-Chlor-, 4-Chlor-, 5-Chlor-, 6-Chlor-, 3,4-Dichlor-, 3,5-Dichlor-, 3,6-Dichlor-, 4,5-Dichlor-, 4,6-Dichlor- und 5,6-Dichlor-2-aminoacetyl-pyridin, 3-Aminoacetyl-pyridin, 2-Chlor-, 4-Chlor-, 5-Chlor-, 6-Chlor-, 2,4-Dichlor-, 2,5-Dichlor-, 2,6-Dichlor-, 4,5-Dichlor-, 4,6-Dichlor- und 5,6-Dichlor-3-aminoacetyl-pyridin, 4-Aminoacetyl-pyridin, 2-Chlor-, 3-Chlor-, 2,3-Dichlor-, 2,5-Dichlor-, 2,6-Dichlor- und 3,6-Dichlor-4-aminoacetyl-pyridin.

Einige Verbindungen der Formel (II) sind bekannt (vgl. J. Chem. Soc. 1938, 753-755; Chem. Pharm. Bull. 32 (1984), 2536-2543), insbesondere die Verbindungen der Formel IIa sind neue.

Das oben unter 1 beschriebene erfindungsgemäße Verfahren - "Verfahren (1)" - wird unter Verwendung von Hydrierungsmitteln durchgeführt. Es können die üblichen zur Hydrierung von Aldehyden oder Ketonen zu Alkoholen geeigneten Reduktionsmittel eingesetzt werden.

Als bevorzugte Hydrierungsmittel für das erfindungsgemäße Verfahren (1) seien genannt:
komplexe Hydride, wie z.B. Lithium-, Natrium- und Kalium-tetrahydridoborat (-borhydrid, -boranat), Lithium-, Natrium- und Kalium-tetrahydridoaluminat (-alanat), Wasserstoff (in Gegenwart von Katalysatoren, vgl. "Reaktionshilfsmittel"),
Alkoholate, wie z.B. Aluminiumisopropylat.

Besonders bevorzugtes Hydrierungsmittel für das erfindungsgemäße Verfahren (1) ist Natriumtetrahydridoborat (Natriumborhydrid, Natriumboranat).

Reaktionshilfsmittel, welche bei Verfahren (1) - insbesondere bei Verwendung von Wasserstoff als Hydrierungsmittel - verwendet werden können, sind vor allem die üblichen Hydrierungskatalysatoren, wie z.B. Raney-Nickel, Palladium und Platin.

Das erfindungsgemäße Verfahren (1) zur Herstellung der Aminoethanolderivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Methyl-tert-butylether, Glykoldimethylether und Diglykoldimethylether, Tetrahydrofuran und Dioxan, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid; ferner auch Alkohole wie Methanol, Ethanol, Propanol, Isopropanol, Butanol, Isobutanol, sec-Butanol, tert-Butanol, Pentanol, Isopentanol, sec-Pentanol und tert-Pentanol sowie auch Wasser.

Die Reaktionstemperaturen können bei dem erfindungsgemäßen Verfahren (1) in einem größeren Bereich variiert werden. Im allgemeinen arbeitet man bei Temperaturen zwischen -20 °C und +200 °C, vorzugsweise bei Temperaturen zwischen 0 °C und 120 °C.

Das erfindungsgemäße Verfahren (1) wird im allgemeinen unter Normaldruck durchgeführt. Es ist jedoch auch möglich, unter erhöhtem oder vermindertem Druck, im allgemeinen zwischen 0,1 und 100 bar, zu arbeiten.

Zur Durchführung des erfindungsgemäßen Verfahrens (1) setzt man auf 1 Mol Aminomethylketon der Formel (II) im allgemeinen zwischen 1 und 5 Mol, vorzugsweise zwischen 1,5 und 3 Mol, Hydrierungsmittel ein.

Zur Durchführung des erfindungsgemäßen Verfahrens (1) können die Reaktionskomponenten in beliebiger Reihenfolge zusammengegeben werden.

In einer bevorzugten Ausführungsform des erfindungsgemäßen Verfahrens (1) wird das Aminomethylketon der Formel (II) in einem geeigneten Verdünnungsmittel und gegebenenfalls zusammen mit einem Reaktionshilfsmittel vorgelegt und das Hydrierungsmittel wird nach Maßgabe der Umsetzungsgeschwindigkeit eindosiert. Das komplette Reaktionsgemisch wird noch bis zum Ende der Umsetzung gerührt und nach üblichen Methoden aufgearbeitet.

Beispielsweise wird mit einer starken Säure, wie z.B. Salzsäure, angesäuert, gegebenenfalls mit einem mit Wasser praktisch nicht mischbaren organischen Lösungsmittel, wie z.B. Methyl-tert-butylether, verdünnt und gut durchgeschüttelt. Dann wird die wäßrige Phase abgetrennt, auf etwa 1/4 bis 1/2 des Volumens eingeengt, mit einer starken Base, wie z.B. Natronlauge, alkalisch gestellt und das hierbei kristallin angefallene Produkt durch Absaugen isoliert.

Die Verbindungen der Formel (IIa) sind neu. In der Formel (IIa) steht die Ketogruppe in 2-, 3- oder 4-Stellung, bevorzugt in 3- oder 4-Stellung, ganz besonders bevorzugt in 4-Stellung des Pyridylrings.

$R^2$ steht bevorzugt für Fluor, Chlor, Brom oder CN, besonders bevorzugt für Chlor.

$R^3$ steht bevorzugt für Wasserstoff, Chlor oder $NH_2$, besonders bevorzugt für Chlor oder $NH_2$ ganz besonders bevorzugt für Chlor.

$R^4$ steht bevorzugt für Wasserstoff, Fluor, Chlor, Brom, besonders bevorzugt für Wasserstoff.

Säureaddukte von Verbindungen der Formel (IIa) sind ebenfalls vorzugsweise die Addukte mit Hydrogenhalogeniden, wie z.B. Hydrogenchlorid, Hydrogenbromid und Hydrogeniodid, insbesondere die Hydrogenchlorid-Addukte.

Die neuen Aminomethylketone der Formel (IIa) können nach dem oben unter 3 beschriebenen erfindungsgemäßen Verfahren - "Verfahren (3)" - hergestellt werden.

Die beim erfindungsgemäßen Verfahren (3) als Ausgangsstoffe zu verwendenden Oxazolcarbonsäureester sind durch die Formel (III) allgemein definiert.

In Formel (III) hat $R^1$ vorzugsweise bzw. insbesondere diejenige Bedeutung, die bereits oben im Zusammenhang mit der Beschreibung der Verbindungen der Formeln (1) und (II) vorzugsweise bzw. als insbesondere bevorzugt für R angegeben wurde und $R^5$ steht vorzugsweise für Methyl, Ethyl, Propyl, Isopropyl, Butyl oder Isobutyl, insbesondere für Methyl oder Ethyl.

Als Beispiele für die Verbindungen der Formel (III) seien genannt:
2-(4-Methoxycarbonyl-5-oxazolyl)-pyridin, 2-(4-Ethoxycarbonyl-5-oxazolyl-)-pyridin, 3-Chlor-, 4-Chlor-, 5-Chlor-, 6-Chlor-, 3,4-Dichlor-, 3,5-Dichlor-, 3,6-Dichlor-, 4,5-Dichlor-, 4,6-Dichlor- und 5,6-Dichlor-2-(4-methoxycarbonyl-5-oxazolyl)-pyridin und -2-(4-ethoxycarbonyl-5-oxazolyl)-pyridin, 3-(4-Methoxycarbonyl-5-oxazolyl)-pyridin, 3-(4-Ethoxycarbonyl-5-oxazolyl)-pyridin, 2-Chlor-, 4-Chlor-, 5-Chlor-, 6-Chlor-, 2,4-Dichlor-, 2,5-Dichlor-, 2,6-Dichlor-, 4,5-Dichlor-, 4,6-Dichlor und 5,6-Dichlor-3-(4-methoxycarbonyl-5-oxazolyl)-pyridin und 3-(4-Ethoxycarbonyl-5-oxazolyl)-pyridin, 4-(4-Methoxycarbonyl-5-oxazolyl)-pyridin, 4-(4-Ethoxycarbonyl-5-oxazolyl)-pyridin, 2-Chlor-, 3-Chlor-, 2,3-Dichlor-, 2,5-Dichlor-, 2,6-Dichlor- und 3,6-Dichlor-4-(4-methoxycarbonyl-5-oxazolyl)-pyridin und -4-(4-ethoxycarbonyl-5-oxazolyl)-pyridin.

Die Ausgangsstoffe der Formel (III) sind teilweise bekannt (vgl. Chem. Abstracts Zitat 110, 23871; Chem. Pharm. Bull. 32 (1984), 2536-2543).

Das oben unter 3 beschriebene erfindungsgemäße Verfahren - "Verfahren (3)" - wird unter Verwendung von Säuren durchgeführt. Geeignete Säuren für Verfahren (3) sind vor allem Protonensäuren, wie z.B. Hydrogenchlorid, Hydrogenbromid und Hydrogeniodid. Hydrogenchlorid wird bei Verfahren (3) vorzugsweise als Säure eingesetzt.

Verfahren (3) wird in Gegenwart eines Verdünnungsmittels durchgeführt. Geeignete Verdünnungsmittel sind neben Wasser vor allem Alkohole, wie z.B. Methanol, Ethanol, Propanol, Isopropanol, Butanol, Isobutanol, sec-Butanol, tert-Butanol, Pentanol, Isopentanol, sec-Pentanol und tert-Pentanol, insbesondere Methanol und Ethanol.

Die Reaktionstemperaturen können bei dem erfindungsgemäßen Verfahren (3) in einem größeren Bereich variiert werden. Im allgemeinen arbeitet man bei Temperaturen zwischen 50 °C und 100 °C, vorzugsweise bei Temperaturen zwischen 70 °C und 95 °C.

Das erfindungsgemäße Verfahren (3) wird im allgemeinen unter Normaldruck durchgeführt. Es ist jedoch auch möglich, unter erhöhtem oder vermindertem Druck, im allgemeinen zwischen 0,1 und 10 bar, zu arbeiten.

Zur Durchführung des erfindungsgemäßen Verfahrens (3) setzt man auf 1 Mol Oxazolcarbonsäureester der Formel (III) im allgemeinen zwischen 1 und 100 Mol, vorzugsweise zwischen 5 und 50 Mol, Säure ein.

Zur Durchführung des erfindungsgemäßen Verfahrens (3) können die Reaktionskomponenten in beliebiger Reihenfolge zusammengegeben werden.

In einer bevorzugten Ausführungsform des erfindungsgemäßen Verfahrens (3) wird der Oxazolcarbonsäureester der Formel (III) vorgelegt und gegebenenfalls mit einem Alkohol verrührt. Dann wird entweder erst die Säure dazugegeben und dann das Gemisch auf die erforderliche Reaktionstemperatur gebracht oder erst aufgeheizt und dann die Säure eindosiert. Das Reaktionsgemisch wird bis zum Ende der Umsetzung gerührt und dann auf Raumtemperatur abgekühlt. Das kristallin angefallene Produkt (Säureaddukt der Verbindung der Formel (II)) kann dann durch Absaugen isoliert werden. Die weitere Reinigung des Produktes kann nach üblichen Methoden durchgeführt werden (vgl. die Herstellungsbeispiele).

Die Verbindungen der Formel (IIIa) sind neu.

In der Formel (IIIa) stehen $R^2$, $R^3$, $R^4$ vorzugsweise für die bei den Verbindungen der Formel IIa angegebenen bevorzugten Reste.

$R^5$ steht insbesondere für Methyl oder Ethyl.

Die Oxazolcarbonsäureester der Formel (IIIa) können also nach dem oben unter 5 beschriebenen erfindungsgemäßen Verfahren - "Verfahren (5)" - hergestellt werden.

Die beim erfindungsgemäßen Verfahren (5) zur Herstellung von Verbindungen der Formel (III) als Ausgangsstoffe zu verwendenden Carbonsäurechloride sind durch die Formel (IV) allgemein definiert.

In Formel (IV) hat $R^1$ vorzugssweise bzw. insbesondere diejenige Bedeutung, die bereits oben im Zusammenhang mit der Beschreibung der Verbindungen der Formeln (I) und (II) vorzugsweise bzw. als insbesondere bevorzugt für $R^1$ angegeben wurde.

Die Carbonsäurechloride der Formel (IV) sind bekannt und/oder können nach an sich bekannten Verfahren hergestellt werden (vgl. EP-A 244 728). Wobei w.U. reaktive Substituenten des Pyridinrings wie $NH_2$ vor der Umsetzung zum Säurechlorid durch geeignete Schutzgruppen wie die Acetylgruppe geschützt werden.

Die beim erfindungsgemäßen Verfahren (5) weiter als Ausgangsstoffe zu verwendenden Isocyanessigsäureester sind durch die Formel (V) allgemein definiert. In Formel (V) hat $R^5$ vorzugsweise bzw.

insbesondere diejenige Bedeutung, die bereits oben im Zusammenhang mit der Beschreibung der Verbindungen der Formel (III) vorzugsweise bzw. als insbesondere bevorzugt für $R^5$ angegeben wurde.

Die Isocyanessigsäureester der Formel (V) sind bekannt und/oder können nach an sich bekannten Verfahren hergestellt werden (vgl. Angew. Chem. 77 (1965), 492-504; Chem. Ber. 108 (1975), 1580-1592).

Das erfindungsgemäße Verfahren (5) zur Herstellung der Oxazolcarbonsäureester der Formel (III) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (5) alle üblicherweise für derartige Umsetzungen verwendbaren Säurebindemittel eingesetzt werden. Vorzugsweise in Frage kommen Alkalimetallhydride wie z. B. Natrium- und Kaliumhydrid, Alkalimetallalkoholate wie Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (5) in einem größeren Bereich variiert werden. Im allgemeinen arbeitet man bei Temperaturen zwischen -20 °C und +50 °C, vorzugsweise bei Temperaturen zwischen -10 °C und +40 °C.

Das erfindungsgemäße Verfahren (5) wird im allgemeinen unter Normaldruck durchgeführt. Es ist jedoch auch möglich, unter erhöhtem oder vermindertem Druck, im allgemeinen zwischen 0,1 und 10 bar, zu arbeiten.

Zur Durchführung des erfindungsgemäßen Verfahrens (5) setzt man auf 1 Mol Carbonsäurechlorid der Formel (IV) im allgemeinen zwischen 0,8 und 1,2 Mol, vorzugsweise zwischen 0,95 und 1,05 Mol, Isocyanessigsäurester der Formel (V) und im allgemeinen zwischen 1 und 2 Mol, vorzugsweise zwischen 1,05 und 1,2 Mol, Säureakzeptor ein.

Zur Durchführung von Verfahren (5) können die Reaktionskomponenten in beliebiger Reihenfolge zusammengegeben werden.

In einer bevorzugten Ausführungsform des erfindungsgemäßen Verfahrens (5) wird das Säurechlorid der Formel (IV) in einem Verdünnungsmittel vorgelegt und der Isocyanessigsäureester der Formel (V) dazugegeben. Dann wird der Säureakzeptor langsam eindosiert und das Reaktionsgemisch bis zum Ende der Umsetzung gerührt. Die Aufarbeitung kann nach üblichen Methoden durchgeführt werden.

Beispielsweise wird filtriert, das Filtrat durch Abdestillieren von Lösungsmittel unter vermindertem Druck auf etwa ein Drittel des Volumens eingeengt und dann durch langsame Zugabe von Wasser auf etwa das vorherige Volumen verdünnt. Das hierbei kristallin anfallende Produkt der Formel (III) kann durch Absaugen isoliert werden.

Bei einem alternativen Aufarbeitungsverfahren wird filtriert, vom Filtrat des Lösungsmittel unter vermindertem Druck abdestilliert und der Rückstand mit Wasser und einem mit Wasser praktisch nicht mischbaren organischen Lösungsmittel, wie z.B. Essigsäureethylester, geschüttelt. Von der organischen Phase wird dann das Lösungsmittel unter vermindertem Druck sorgfältig abdestilliert. Der verbleibende Rückstand enthält im wesentlichen das Produkt der Formel (III).

Die nach dem erfindungsgemäßen Verfahren herzustellenden Aminoethanolderivate der Formel (I) können als Zwischenprodukte zur Herstellung von Arzneimitteln (vgl. EP-A 305 845) oder von Leistungsförderen bei Tieren (vgl. DE-P 3 902 286 vom 26.01.1989) verwendet werden.

<u>Herstellungsbeispiele:</u>

<u>Beispiel 1</u>

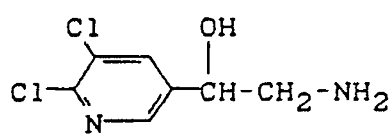

Eine Lösung von 0,9 g (24 mMol) Natriumboranat in 3 ml Wasser wird unter Rühren zu einer auf 0° C bis 5° C abgekühlten Mischung aus 2,85 g (12 mMol) 5,6-Dichlor-3-aminoacetyl-pyridin-hydrochlorid und 30 ml Methanol tropfenweise gegeben. Das Reaktionsgemisch wird 8 Stunden bei 0° C bis 5° C gerührt und anschließend im Wasserstrahlvakuum eingeengt. Zum Rückstand werden 50 ml Wasser, 2 ml Essigsäure und 50 ml Essigsäureethylester gegeben und das Gemisch wird gut durchgerührt. Dann werden die Phasen getrennt, die wäßrige Phase mit konzentrierter Natronlauge auf pH>12 eingestellt, durchgerührt und mit Eis gekühlt. Das hierbei kristallin angefallene Produkt wird durch Absaugen isoliert.

Man erhält 1,5 g (61% der Theorie) 5,6-Dichlor-3-(2-amino-1-hydroxy-ethyl)-pyridin vom Schmelzpunkt 205-208° C.

Beispiel 2

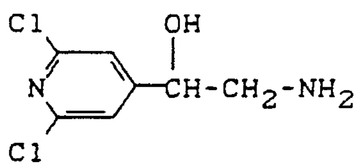

Eine Lösung von 5,7 g (0,15 Mol) Natriumboranat in 25 ml Wasser wird unter Rühren zu einer auf 0° C bis 5° C gekühlten Mischung aus 18,1 g (0,075 Mol) 2,6-Dichlor-4-aminoacetyl-pyridin-hydrochlorid, 150 ml Wasser und 25 ml Methyl-tert-butylether innerhalb von 40 Minuten tropfenweise gegeben. Das Reaktionsgemisch wird 4 Stunden bei 0° C bis 5° C gerührt, dann tropfenweise mit 13 ml konzentrierter Salzsäure und 50 ml Methyl-tert-butylether versetzt und geschüttelt. Anschließend werden die Phasen getrennt, die wäßrige Phase auf etwa ein Viertel des ursprünglichen Volumens eingeengt, mit 26 ml 5N-Natronlauge auf pH 12 eingestellt und gerührt. Das kristallin angefallene Produkt wird durch Absaugen isoliert.

Man erhält 13,3 g (86% der Theorie) 2,6-Dichlor-4-(2-amino-1-hydroxy-ethyl)-pyridin vom Schmelzpunkt 145° C.

Zwischenprodukte der Formel (II)

Beispiel (II-1)

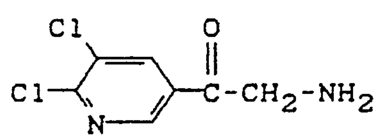 x HCl

Eine Mischung aus 4,3 g (15 mMol) 5,6-Dichlor-3-(4-ethoxycarbonyl-5-oxazolyl)-pyridin und 20 ml Ethanol wird auf 80° C erhitzt und bei dieser Temperatur tropfenweise mit 13,5 g konzentrierter Salzsäure versetzt. Das Reaktionsgemisch wird 10 Stunden unter Rückfluß zum Sieden erhitzt, dann eingeengt, mit 10 ml Eiswasser verrührt und abgesaugt.

Man erhält 2,85 g (79% der Theorie) 5,6-Dichlor-3-aminoacetyl-pyridin-hydrochlorid, welches oberhalb von 280° C unter Zersetzung schmilzt.

Beispiel (II-2)

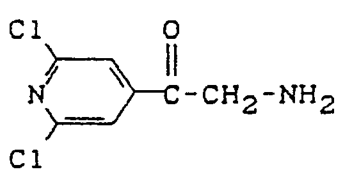 x HCl

Eine Mischung aus 14,35 g (0,05 Mol) 2,6-Dichlor-4-(4-ethoxycarbonyl-5-oxazolyl)-pyridin und 150 ml konzentrierter Salzsäure wird langsam (Schaumentwicklung!) auf 80° C aufgeheizt und bei dieser Temperatur 7 Stunden gerührt. Nach Abkühlen wird das kristalline Produkt durch Absaugen abgetrennt, der Rückstand in 150 ml Wasser aufgenommen und filtriert. Die beiden Filtrate werden vereinigt und mit 300 ml Toluol geschüttelt. Die organische Phase wird abgetrennt, mit Aktivkohle geklärt und filtriert. Vom Filtrat wird das Lösungsmittel im Wasserstrahlvakuum sorgfältig abdestilliert.

Man erhält 10,0 g (83% der Theorie) 2,6-Dichlor-4-aminoacetyl-pyridin-hydrochlorid als kristallinen Rückstand vom Schmelzpunkt 232° C-234° C.

Beispiel (III-3)

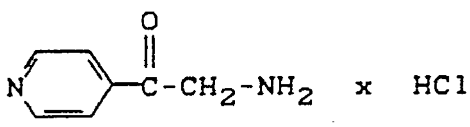

4-Aminoacetyl-pyridin-hydrochlorid (Schmelzpunkt: 268° C-270° C/Zersetzung) wird analog zu den Beispielen (II-1) und (II-2) erhalten.

Zwischenprodukte der Formel (III)

Beispiel (III-1)

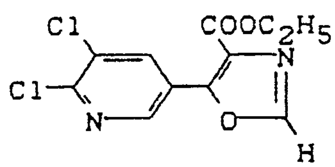

Zu einer Mischung aus 15,8 g (75 mMol) 5,6-Dichlor-isonicotinsäurechlorid und 100 ml Tetrahydrofuran werden bei 20° C unter Rühren 8,5 g (75 mMol) Isocyanessigsäureethylester und dann 8,3 g (82 mMol) Triethylamin tropfenweise gegeben. Das Reaktionsgemisch wird 17 Stunden bei 20° C gerührt und dann eingeengt. Der Rückstand wird mit 400 ml Wasser/Essigsäureethylester (Vol. 1/1) geschüttelt, die organische Phase dann abgetrennt und eingeengt.

Man erhält 17,8 g (83% der Theorie) 5,6-Dichlor-3-(4-ethoxycarbonyl-5-oxazolyl)-pyridin als kristallinen Rückstand vom Schmelzpunkt 95° C-98° C.

Beispiel (III-2)

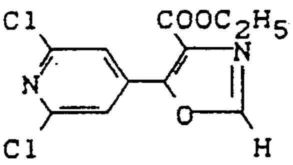

Zu einer Mischung aus 210,5 g (1,0 Mol) 2,6-Dichlor-pyridin-4-carbonsäurechlorid und 1,5 l Tetrahydrofuran werden bei 0° C bis 5° C 113 g (1,0 Mol) Isocyanessigsäureethylester innerhalb von 30 Minuten tropfenweise gegeben und das Gemisch wird 45 Minuten bei 5° C gerührt. Dann werden bei dieser Temperatur 111 g (1,1 Mol) Triethylamin zugetropft und das Gemisch wird 20 Stunden bei 20° C gerührt.

Dann wird filtriert, das Filtrat auf ca. 500 ml eingeengt und unter Rühren werden ca. 2000 ml Wasser

langsam zugetropft. Das hierbei kristallin angefallene Produkt wird durch Absaugen isoliert.

Man erhält 267 g (93% der Theorie) 2,6-Dichlor-4-(4-ethoxycarbonyl-5-oxazolyl)-pyridin vom Schmelzpunkt 108° C-109° C.

Beispiel (III-3)

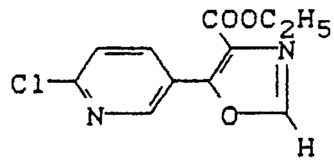

6-Chlor-3-(4-ethoxycarbonyl-5-oxazolyl)-pyridin (Schmelzpunkt 77° C-79° C) wird analog zu den Beispielen (III-1) und (III-2) erhalten.

Beispiel (III-4)

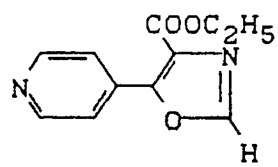

4-(4-Ethoxycarbonyl-5-oxazolyl)-pyridin (Schmelzpunkt 51° C-53° C) wird analog zu den Beispielen (III-1) und (III-2) erhalten.

**Patentansprüche**

**1.** Verfahren zur Herstellung von Aminoethanolderivaten der allgemeinen Formel (I)

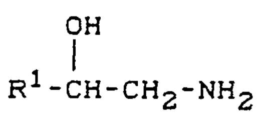 (I)

in welcher

$R^1$ für gegebenenfalls substituiertes Pyridyl steht,

dadurch gekennzeichnet, daß man Aminomethylketone der allgemeinen Formel (II)

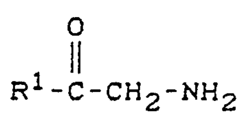 (II)

in welcher

$R^1$ die oben angegebene Bedeutung hat,

oder Säureaddukte von Aminomethylketonen der Formel (II) mit Hydrierungsmitteln gegebenenfalls in Gegenwart von Reaktionshilfsmitteln und gegebenenfalls in Gegenwart von Verdünnungsmitteln bei Temperaturen zwischen -50° C und +150° C umsetzt;

**2.** Pyridylaminomethylketone der allgemeinen Formel (IIa)

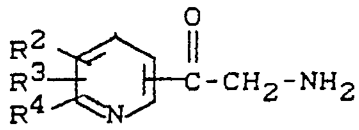

(IIa)

in welcher

$R^2$ für Halogen oder CN steht,

$R^3$ für H, Halogen oder $NH_2$ steht,

$R^4$ für H oder Halogen steht

und deren Säureaddukte.

**3.** Verfahren zur Herstellung von Aminomethylketonen der allgemeinen Formel (II)

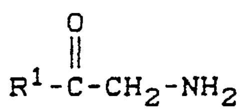

(II)

in welcher

$R^1$ für gegebenenfalls substituiertes Pyridyl steht,

oder von Säureaddukten von Aminomethylketonen der Formel (II)

dadurch gekennzeichnet, daß man Oxazolcarbonsäureester der allgemeinen Formel (III)

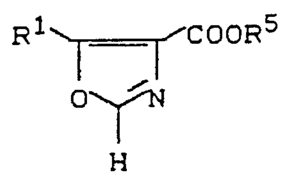

(III)

in welcher

$R^1$ die oben angegebene Bedeutung hat und

$R^5$ für Niederalkyl steht,

mit Säuren bei Temperaturen zwischen 50° C und 100° C in Gegenwart eines Verdünnungsmittels umsetzt, und gegebenenfalls die Produkte anschließend durch Behandeln mit starken Basen freisetzt.

**4.** Oxazolcarbonsäureester der allgemeinen Formel (IIIa)

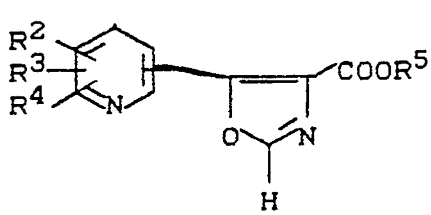

(IIIa)

in welcher

$R^2$, $R^3$, $R^4$ die in Anspruch 2 angegebene Bedeutung haben.

**5.** Verfahren zur Herstellung von Oxazolcarbonsäureestern der allgemeinen Formel (IIIa)

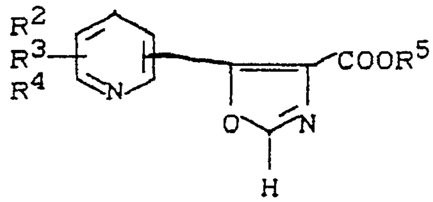

(III)

in welcher

$R^2$, $R^3$, $R^4$, $R^5$ die in Anspruch 4 angegebene Bedeutung haben,

dadurch gekennzeichnet, daß man Carbonsäurechloride der allgemeinen Formel (IV)

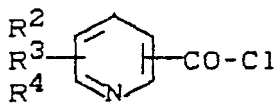

(IV)

in welcher

$R^2$, $R^3$, $R^4$ die oben angegebene Bedeutung haben, mit Isocyanessigsäureestern der allgemeinen Formel (V)

$CN-CH_2-COOR^5$ (V)

in welcher

$R^5$ die oben angegebene Bedeutung hat

in Gegenwart eines Säureakzeptors und gegebenenfalls in Gegenwart eines Verdünnungsmittels bei Temperaturen zwischen -20° C und +50° C umsetzt.